# EUROPÄISCHE PATENTSCHRIFT

(11) **EP 3 279 150 B1**
(45) Veröffentlichungstag und Bekanntmachung des Hinweises auf die Patenterteilung: **03.07.2019**
(21) Anmeldenummer: 16001699.4
(22) Anmeldetag: 02.08.2016
(51) Int. Cl.: C02F 1/32, H01J 3/00, A61L 2/10, H01J 5/48, H01J 61/34, B01J 19/12

(54) **STRAHLEREINHEIT MIT HALTERUNG UND KOPFTEIL**
RADIATION UNIT WITH RETAINER AND HEAD
UNITE D'EMETTEUR COMPRENANT UNE FIXATION ET UN ELEMENT DE TETE

(43) Veröffentlichungstag der Anmeldung: 07.02.2018
(73) Patentinhaber: Peschl Ultraviolet GmbH, 55130 Mainz (DE)
(72) Erfinder: Peschl, Alexander, 55130 Mainz (DE); Peschl, Günther, 55130 Mainz (DE)
(74) Vertreter: mepat Patentanwälte

(56) Entgegenhaltungen:
- EP-A1- 0 555 536
- WO-A1-2007/071042
- WO-A1-2007/139095
- WO-A1-2014/170788
- US-A- 3 249 781

## Beschreibung

Die Erfindung betrifft eine Strahlereinheit mit einer Halterung und einem Kopfteil für einen Strahler.

Aus dem Stand der Technik ist bekannt, zur elektrischen Versorgung von Strahlern Kopfteile einzusetzen, die nicht nur für den elektrischen Anschluss sorgen, sondern auch die Halterung des Strahlers bilden.

In DE 10 2013 006 635 A1 wird eine Strahlereinheit aus UV-Strahler und Kopfteil beschrieben, die in einem Modul von einem Rahmen gehalten wird. Das Kopfteil hat einen Verlängerungsadapter, an dessen einem Ende der Strahler angeschlossen wird und der an seinem anderen Ende ein umgebendes Steckerelement für den elektrischen Anschluss an einer zylindrischen Buchse aufweist. Die Buchse ist durch eine Überwurfmutter an dem Steckerelement befestigt, das seinerseits in einem kegelförmigen Kopfstück aufgenommen und mittels einer weiteren Überwurfmutter daran befestigt ist. Der Verlängerungsadapter kann abhängig von dem gewünschten Modulaufbau in beliebiger Länge gewählt werden. Eine Schutzgaszufuhr ist dabei nicht vorgesehen.

Weiter ist bekannt, dass Kopfteile zur Zufuhr (und Abfuhr) von Schutzgas genutzt werden können. EP 2 674 208 A1 offenbart ein becherartiges Kopfteil, das über einen Ringflansch mit einem Hüllrohr, das den Strahler umgibt, verbunden wird. Das Kopfteil weist eine axial-zentrale Öffnung für ein Anschlussstück für den elektrischen Anschluss des Strahlers und eine Zu- und Abfuhreinrichtung für Schutzgas auf.

EP 0 555 536 A1 offenbart eine Tauchlampe mit einem Außenkolben, in dem eine Entladungslampe mit zwei sich gegenüberliegenden Steckanschlüssen angeordnet ist, von denen ein Anschluss in eine als federbelastetes Gleitteil ausgebildete Fassung zwecks Kontaktierung eingreift. Der andere Anschluss ist mit einer von zwei rohrförmigen Führungsstangen gehaltenen Fassung elektrisch kontaktierend und mechanisch lösbar verbunden, sodass die Führungsstangen zum einen als Stromzuleiter zum anderen als Inertgaszufuhr dienen. Die Führungsstangen sind im Anschlusskopf in einer Zwischenplatte aufgenommen, mit dem die Strahlereinheit an dem Außenkolben befestigt ist. Die Zwischenplatte teilt den Anschlusskopf in einen Inertgaszuführraum oberhalb der Zwischenplatte, in den sich die rohrförmigen Führungsstangen zur Zufuhr des Schutzgases und zum elektrischen Ansschluss erstrecken, und in einen Gasauslassraum unterhalb der Zwischenplatte, der mit dem Innenraum des Außenkolbens verbunden ist.

Ausgehend von diesem Stand der Technik ist es Aufgabe der vorliegenden Erfindung, eine verbesserte Strahlereinheit mit Halterung und Kopfteil bereitzustellen, die nicht nur eine Schutzgaszufuhr sondern auch eine Längenanpassung der Halterung mit elektrischem Anschluss des Strahlers ermöglicht, und die zugleich eine einfache und sichere Montage und exakte Positionierbarkeit des Strahlers in einem Hüllrohr sicherstellt.

Diese Aufgabe wird durch eine Strahlereinheit mit den Merkmalen des Anspruchs 1 gelöst. Weiterbildungen der Strahlereinheit sind in den Unteransprüchen ausgeführt.

Eine erfindungsgemäße Strahlereinheit setzt sich aus einem Kopfteil und einer Halterung für einen Strahler zusammen, wobei das Kopfteil einen strahlerfernen Grundkörper aus einem elektrisch isolierenden Material mit einer elektrischen Anschlusseinrichtung und einer Schutzgaszufuhreinrichtung aufweist. Als Halterung für den Strahler ist ein Gestänge vorgesehen, das aus einer Mehrzahl Rohre oder aus einer Mehrzahl Stangen und zumindest einem Rohr aus einem elektrisch leitenden Material besteht. Die für den Strahler erforderlichen elektrischen Anschlussleitungen werden dabei durch zumindest einen Teil der Rohre oder Stangen gebildet, die dazu einenends mit der elektrischen Anschlusseinrichtung in Kontakt stehen und an ihrem anderen Ende den elektrischen Anschluss des Strahlers bereitstellen. Zudem steht zumindest eines der Rohre einenends mit der Schutzgaszufuhreinrichtung in fluidischer Kommunikation und sorgt damit anderenends für die Schutzgaszufuhr zum Strahler. Erfindungsgemäß weist das Kopfteil der Strahlereinheit einen strahlernahen Anschlusskörper auf, der lösbar mit dem Grundkörper verbindbar bzw. verbunden ist. Dabei weist der Anschlusskörper parallel orientierte Durchtrittsöffnungen für das Gestänge auf, das die Halterung für den Strahler bildet und das an dem Anschlusskörper lösbar befestigt ist. Der Grundkörper wiederum weist Bohrungen auf, die mit den Durchtrittsöffnungen im Anschlusskörper fluchten, so dass die Bohrungen eine Schnittstelle des Kopfteil-Grundkörpers, der eine elektrische Anschlusseinrichtung und eine Schutzgaszufuhreinrichtung aufweist, zu dem im Anschlusskörper aufgenommenen Gestänge bereitstellen. Grund- und Anschlusskörper sind aus einem elektrisch isolierenden Material, beispielsweise aus einem temperatur- und vorzugsweise auch UVstabilen Kunststoff beschaffen. Das Gestänge wird aus Rohren oder aus Stangen, zumindest aber mit einem Rohr, falls die weiteren Gestängekomponenten Stangen sind, aus einem elektrisch leitenden Material gebildet. Die für den Strahler erforderlichen elektrischen Anschlussleitungen werden durch zumindest einen Teil der Rohre bzw. der Stangen und des zumindest einen Rohrs gebildet. Die Anzahl der Rohre bzw. Rohre und Stangen, die das Gestänge bilden, kann dabei die Anzahl der für den Strahler erforderlichen elektrischen Anschlussleitungen übersteigen. Der elektrische Kontakt derjenigen Rohre bzw. Rohre und Stangen, die die elektrischen Anschlussleitungen für den Strahler bereitstellen, mit der elektrischen Anschlusseinrichtung, wird durch die Verbindung des Grundkörpers mit dem Anschlusskörper bereitgestellt, d. h. wenn der Grundkörper und der Anschlusskörper verbunden sind. Entsprechend wird die fluidische Kommunikation des zumindest einen Rohrs, das zur Schutzgaszufuhr zum Strahler vorgesehen ist, mit der Schutzgaszufuhreinrichtung durch die Verbindung des Grundkörpers mit dem Anschlusskörper bereitgestellt, sodass das zumindest eine Rohr einenends mit der Schutzgaszufuhreinrichtung in fluidischer Kommunikation (kann also ein Fluid von der Schutzgaszufuhreinrichtung übernehmen und führen) steht, wenn der Grundkörper und der Anschlusskörper verbunden sind, wobei dieses zumindest eine Rohr an seinem anderen Ende, also dem vom Kopfteil am weitesten entfernt liegenden Punkt, die Schutzgaszufuhr zum Strahler bereitstellt.

Zur Vereinfachung wird im Folgenden nur von "Rohren" des Gestänges gesprochen, das in einer bevorzugten Ausführungsform auch aus ausschließlich Rohren bestehen kann. Es wird aber darauf hingewiesen, dass unter den beschriebenen Ausführungsformen auch Gestänge aus Stangen und zumindest einem Rohr verstanden werden sollen.

Zu den Bohrungen im Grundkörper, die Sackbohrungen mit einem dem Anschlusskörper zugewandten offenen Ende sein können, erstrecken sich durch den Grundkörper Verbindungsbohrungen von zumindest einer der Seiten des Grundkörpers in einem von dem Anschlusskörper entfernt liegenden Bereich des Grundkörpers. In diesen Verbindungsbohrungen liegt für jede der Anschlussleitungen (üblicherweise zumindest eine Phase und ein Neutralleiter, gegebenenfalls auch ein Schutzleiter), die für den Strahler erforderlich sind, jeweils ein elektrisches Kontaktelement der elektrischen Anschlusseinrichtung vor. Dieses kontaktiert eine Steckhülse, die in den Grundkörper-Bohrungen angeordnet sind. Diese Steckhülsen sind direkt oder über einen Verbindungsstecker mit jeweils einem der Rohre verbunden, die in den Durchtrittsöffnungen des Anschlusskörpers aufgenommen sind.

Um Schutzgas zuzuführen, ist vorgesehen, dass zumindest eine der Bohrungen im Grundkörper auf der dem Anschlusskörper zugewandten Seite von einer Sackringbohrung konzentrisch umgeben ist. Unter Sackringbohrung wird eine ringförmige Bohrung verstanden, die ein geschlossenes Ende aufweist. Die Tiefe der Sackringbohrung ist kleiner als ein Abstand zu den Verbindungsbohrungen, die in einem von dem Anschlusskörper entfernten Bereich des Grundkörpers liegen und zu den Bohrungen, nicht aber zu der Sackringbohrung führen. Hierfür ist zumindest eine weitere Verbindungsbohrung vorgesehen, die von zumindest einer der Seiten des Grundkörpers in die Sackringbohrung mündet. Diese Verbindungsbohrung zu der Sackringbohrung verläuft vorzugsweise in einem dem Anschlusskörper nahen Bereich des Grundkörpers entsprechend der Tiefe der Sackringbohrung. Vorzugsweise erstrecken sich die Verbindungsbohrungen zu den Bohrungen und die Verbindungsbohrungen zu den Sackringbohrungen von unterschiedlichen Seiten des Grundkörpers aus. Die Verbindungsbohrung zu der Sackringbohrung stellt mit der Sackringbohrung die Schutzgaszufuhreinrichtung in dem Grundkörper des Kopfteils zu dem zumindest einen Rohr, das zur Schutzgaszufuhr zum Strahler vorgesehen ist, bereit.

Die Steckhülsen, die in Grundkörper-Bohrungen angeordnet sind, weisen einen Hülsenabschnitt, dessen offenes Ende in Richtung Anschlusskörper weist, und einen Kontaktabschnitt, der sich in der Bohrung zumindest bis zur Höhe der Verbindungsbohrung erstreckt, auf, so dass das dort befindliche Kontaktelement den Kontaktabschnitt kontaktieren kann. Der Verbindungsstecker, der den Kontakt zwischen der Steckhülse und einem Rohr vermittelt, weist einen Aufnahmeabschnitt für das Rohr und einen Zapfenabschnitt auf, der in den Hülsenabschnitt der Steckhülse aufnehmbar oder aufgenommen ist, so dass der elektrische Kontakt von der Anschlusseinrichtung zu den Rohren des Gestänges einfach beim Zusammenfügen des Grundkörpers mit dem Anschlusskörper des Kopfteils hergestellt wird.

Weiter kann dazu vorgesehen sein, dass sich in dem Anschlusskörper die Durchtrittsöffnung zum Grundkörper hin abgestuft aufweitet, wobei eine erste abgestufte Öffnung, die einen Absatz um die Durchtrittsöffnung bereitstellt, zur Aufnahme des Aufnahmeabschnitts des Verbindungssteckers mit einem entsprechenden Querschnitt ausgebildet ist. Die Tiefe dieses Absatzes ist so gewählt, dass der Zapfenabschnitt des Verbindungssteckers über den Anschlusskörper hinausragen kann, um mit dem Hülsenabschnitt der Steckhülse im Grundkörper in Eingriff treten zu können, wenn Anschlusskörper und Grundkörper verbunden werden. Eine zweite abgestufte Öffnung um die Durchtrittsöffnung im Anschlusskörper ist noch weiter aufgeweitet, so dass deren Durchmesser dem Außendurchmesser der Sackringbohrung im Grundkörper entspricht und damit fluchtet, wenn der Grundkörper und der Anschlusskörper verbunden sind. Die Sackringbohrung im Grundkörper stellt dann zusammen mit der zweiten abgestuften Öffnung im Anschlusskörper einen Schutzgaszufuhrraum dar, aus dem das Gas in das in der jeweiligen Durchtrittsöffnung angeordnete Rohr gelangt.

Hierfür kann der Aufnahmeabschnitt des Verbindungssteckers radiale Verbindungsbohrungen zu einem im Verbindungsstecker vorliegenden Hohlraum aufweisen, der mit einer Mündung des Rohrs, das in dem Aufnahmeabschnitt aufgenommen ist, verbunden ist.

Die lösbare Verbindung des Grundkörpers mit dem Anschlusskörper kann beispielsweise durch Stifte, Schrauben oder Stehbolzen hergestellt werden, die durch entsprechende Bohrungen in Grund- und Anschlusskörper geführt und durch ein geeignetes Sicherungselement wie eine Mutter oder einen Splint gehalten werden. Es sind aber auch alternative Befestigungslösungen wie beispielsweise Klammern, Rastelemente, gegebenenfalls in Kombination mit Pass- bzw. Formelementen an den zugewandten Flächen des Grundkörpers und Anschlusskörpern nach dem Schlüssel-Schloss-Prinzip, die die korrekte Lage der beiden Kopfteilkörper zueinander sicherstellen. Werden Stehbolzen verwendet, so können diese in entsprechenden Bohrungen, insbesondere Sackbohrungen des Anschlusskörpers festgelegt sein, wobei ihre Länge so gewählt ist, dass sie den Grundkörper durchdringen können, der entsprechende mit den Öffnungen fluchtende Durchtrittsöffnungen aufweist. Auf der von dem Anschlusskörper abgewandten Seite des Grundkörpers werden geeignete Sicherungselemente mit den Stehbolzen in Eingriff gebracht. Sind Muttern als Sicherungselemente vorgesehen, können die Durchtrittsöffnungen für die Stehbolzen auf der von dem Anschlusskörper abgewandten Seite des Grundkörpers eine aufgeweitete Stufe aufweisen, so dass die Mutter, die in Eingriff mit dem Stehbolzen kommt, versenkt angeordnet ist und nicht aus dem Grundkörper herausragt.

Die Rohre bzw. Stangen, die das Gestänge bilden, können auch Rohranordnungen aus mehreren aneinandergereihten Rohren bzw. Stangenanordnungen aus mehreren aneinandergereihten Stangen bestehen. Auch wenn im vorliegenden Text bislang "Rohre" bzw. "Stangen" zur Bildung des Gestänges genannt wurden, sollen hierunter auch Rohranordnungen bzw. Stangenanordnungen verstanden werden. Ob Rohre oder Rohranordnungen zur Halterung des Strahlers eingesetzt werden, hängt in erster Linie von der gewünschten Länge der Halterung ab. Rohranordnungen sind bei größerer Länge der Haltung stabiler.

Zur Bildung der Rohranordnung können die Rohre (für eine Stangenanordnung gilt entsprechendes) an den Enden miteinander korrespondierende Eingriffselemente aufweisen. Dabei kann es sich vorzugsweise einenends um einen Außengewindeabschnitt und anderenens um einen korrespondierenden Innengewindeabschnitt handeln, sodass eine gewünschte Länge der Halterung mit einer Rohranordnung durch Verbinden, vorzugsweise Verschrauben mehrerer Rohre, die zudem unterschiedliche Länge aufweisen können, einstellbar ist.

Um einen Überschlag zwischen den als elektrischen Leitern dienenden Rohren zu vermeiden, kann jedes Rohr des Gestänges von einem Isolatorrohr umgeben sein, das beispielsweise aus Quarzglas gefertigt sein kann. Um die Isolatorrohre an die Länge der Gestängerohre anzupassen, kann das Isolatorrohr einen langen Rohrabschnitt mit einem Durchmesser zur Aufnahme des Leiterrohrs und einen endständigen aufgeweiteten Hülsenabschnitt aufweisen, in den das nicht-aufgeweitete Ende des Rohrabschnitts eines weiteren Isolatorrohrs aufgenommen werden kann, so dass in Abhängigkeit der gewünschten Länge der Halterung mehrere Isolatorrohre zu einer Isolatorrohranordnung zusammensteckbar sind.

Das Gestänge einer erfindungsgemäßen Strahlereinheit kann in Abhängigkeit der Länge der Halterung einen oder mehrere Stabilisierungsvorrichtungen, die vorliegend Isoliersterne genannt werden, aufweisen, die über die Länge der Halterung verteilt angeordnet sind, wobei der Isolierstern parallel orientierte Hülsen entsprechend der Anzahl der parallelen Rohre des Gestänges aufweist, und die Hülsen einen Innendurchmesser zur Aufnahme des Rohrabschnitts des Isolatorrohrs aufweisen, das das jeweilige Leiterrohr umgibt. Die Hülsen werden durch ein Verbindungsstück in einer für das Gestänge vorbestimmten Anordnung und Abstand voneinander gehalten. Die Isoliersterne können ebenfalls aus Quarzglas gefertigt sein.

Weiter kann eine erfindungsgemäße Strahlereinheit zumindest zwei Zentrierelemente aufweisen, die für eine zentrierende Halterung des Strahlers in einem Hüllrohr sorgen. Das Zentrierelement weist einen Scheibengrundkörper mit zumindest einer axial-zentralen Bohrung für einen Strahlersockel und zumindest zwei, bevorzugt zumindest drei, exzentrischen Bohrungen für das Gestänge auf. Der Durchmesser des Scheibengrundkörpers ist kleiner als ein Innendurchmesser des Hüllrohrs, und das Zentrierelement weist zumindest ein elastisches Federelement auf, das sich zumindest abschnittsweise an einem Außenumfang des Scheibengrundkörpers erstreckt und eine radiale Rückstellkraft bereitstellt, so dass das Federelement beim Einführen in das Hüllrohr radial komprimiert wird und dadurch zentriert wird. Gleichzeitig bewirkt das Federelement einen gewissen Halt im Hüllrohr, gestattet aber eine axiale Verschiebung beim Einführen des Gestänges mit dem Strahler in das Hüllrohr.

Es kann vorgesehen sein, dass jeweils zumindest ein Zentrierelement beiderseits des Strahlers angeordnet ist, also eines oberhalb und eines unterhalb des Strahlers. In Abhängigkeit der Länge der Halterung können gegebenenfalls noch weitere Zentrierelemente entlang des Gestänges angeordnet sein.

Dabei sorgen die Zentrierelemente nicht nur für zentrierende Halterung, sondern auch für den elektrischen Anschluss des Strahlers. Hierbei erstreckt sich ein erstes der Rohre, das einen Phasenleiter für den Wechselstromanschluss bereitstellt, bis zu dem Zentrierelement, das kopfteilseitig, üblicherweise oberhalb und benachbart zu dem Strahler angeordnet ist, zum elektrischen Anschluss des Strahlers an dem ersten, kopfteilnahen Strahlersockel. Die weiteren Rohre des Gestänges dienen dem Potentialausgleich (Neutralleiter) und erstrecken sich weiter neben dem Strahler bis zu dem Zentrierelement, das kopfteilfern, also unten benachbart zu dem Strahler angeordnet ist. In der axial-zentralen Bohrung dieses kopfteilfernen Zentrierelements liegt ein Anschlusselement für den zweiten, kopfteilfernen Strahlersockel vor, so dass für die axial-zentrierte Anordnung des Strahlers gesorgt ist. Weiter ist das Anschlusselement mit den Rohren, die die Neutralleiter bereitstellen, elektrisch kontaktierend verbunden.

Dabei kann der elektrische Anschluss des ersten Rohrs, das die stromführende Leitung bereitstellt, mit dem ersten, kopfteilnahen Strahlersockel durch eine isolierte Leitung bereitgestellt werden, die Überlänge in Bezug auf den Abstand des Rohrendes des ersten Rohrs zum ersten, kopfteilnahen Strahlersockel aufweist, um thermische Ausdehnungseffekte kompensieren zu können. Ferner kann gegebenenfalls ein zusätzlicher Isolierstern vorgesehen sein, der (zumindest) zwei Hülsen für die weiteren Rohre, die sich bis zu dem unteren, kopfteilfernen Zentrierelement erstrecken, und eine axial-zentrierte Durchtrittsöffnung in dem Verbindungsstück aufweist, in der der erste, kopfteilnahe Strahlersockel aufgenommen und gestützt wird.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine Seitenansicht einer erfindungsgemäßen Strahlereinheit aus Strahler und Halterung mit erfindungsgemäßem Kopfteil aus Grund- und Anschlusskörper,
- **Fig. 2**: eine Seitenansicht der Strahlereinheit aus Fig. 1 ohne Grundkörper, nur mit Anschlusskörper,
- **Fig. 3**: eine Detailseitenansicht auf das untere Ende der Strahlereinheit,
- **Fig. 4**: eine Detailseitenansicht auf das obere Ende des von der Halterung gehaltenen Strahlers,
- **Fig. 5**: eine Draufsicht auf ein Zentrierelement der Halterung,
- **Fig. 6**: eine Untenansicht des unteren, den Strahler zentrierenden Zentrierelements mit Kontaktanschlüssen,
- **Fig. 7**: eine Untenansicht des oberen Zentrierelements mit elektrischem Anschluss für den Strahler,
- **Fig. 8**: eine Querschnittansicht durch das Zentrierelement aus Fig. 6 gemäß Schnittlinie AA,
- **Fig. 9**: eine perspektivische Ansicht auf ein Hüllrohr,
- **Fig. 10**: eine perspektivische Ansicht auf einen Isolierstern,
- **Fig. 11**: eine Seitenansicht auf das Kopfteil aus Grund- und Anschlusskörper,
- **Fig. 12**: eine Seiten-Teilschnittansicht auf das Kopfteil aus Fig. 11,
- **Fig. 13**: eine perspektivische Untenansicht auf den Grundkörper,
- **Fig. 14**: eine Seitenansicht auf den Grundkörper,
- **Fig. 15**: eine Untenansicht auf den Grundkörper,
- **Fig. 16**: eine Draufsicht auf den Anschlusskörper,
- **Fig. 17**: eine Seiten-Teilschnittansicht auf den Anschlusskörper.

Die erfindungsgemäße Vorrichtung ist eine Strahlereinheit aus Kopfteil und Halterung für einen Strahler. Die Strahlereinheit bzw. zumindest das Gestänge mit dem Strahler ist dazu geeignet, in einem Hüllrohr, insbesondere senkrecht in einem Tauchrohr mit einseitig geschlossenem Ende, betrieben zu werden. Der Betrieb in senkrechter Anordnung bietet verschiedene Vorteile - so kann beispielsweise keine Gasakkumulation im Strahlerbereich stattfinden. Ferner ist kein Siphon erforderlich und weder Strahler noch Hüllrohr unterliegen einer Durchbiegungsbelastung, auch nicht bei sehr langen Strahlereinheiten. Die Strahlereinheit im Tauchrohr gestattet auch den Einbau in Rührkesselreaktoren und erlaubt mit nur einem Kopfteil eine einfache Begasung, wobei die Anzahl erforderlicher Dichtungen reduziert werden kann. Über das Kopfteil und die Halterung wird nicht nur der elektrische Anschluss hergestellt, sondern auch eine Inert- bzw. Schutzgaszufuhr ermöglicht, beispielsweise mit Stickstoff, sodass, wenn die Strahlereinheit in einem Tauchrohr angeordnet ist, Explosionsschutz der Zündschutzart "p" durch Überdruckkapselung ermöglicht wird. Hierdurch kann die Strahlereinheit auch in Photoreaktoren zur Durchführung von photochemischen Reaktionen, wie z. B. in der Synthese, eingesetzt werden, die in Lösungsmitteln stattfinden, die potentiell ein Explosionsrisiko bergen. Dies kann durch die erfindungsgemäße Strahlereinheit zuverlässig minimiert werden.

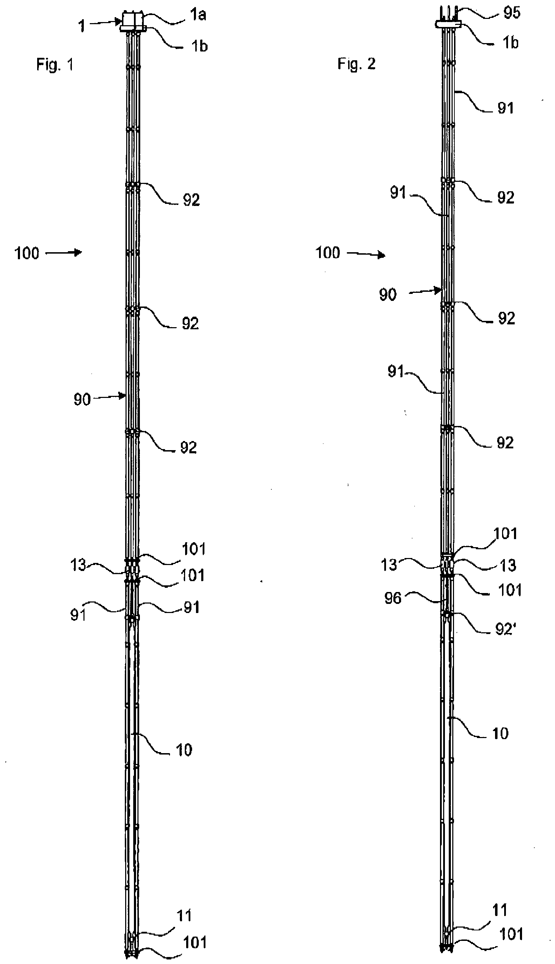

**Fig. 1** zeigt eine erfindungsgemäße Strahlereinheit 100, mit einem Kopfteil 1 und einem Gestänge 90, das die Halterung für einen Strahler 10 bildet. Das Kopfteil 1 besteht aus einem Grundkörper 1a und einem Anschlusskörper 1b, die, wie aus Fig. 2 zu entnehmen ist, über Stehbolzen 95 lösbar miteinander verbunden werden können. Die Stehbolzen 95 können im Anschlusskörper 1b befestigt sein.

Im Kopfteil 1, dessen Grundkörper 1a und Anschlusskörper 1b quasi ein Steckverbindersystem bilden, sind Elemente für den Hochspannungsanschluss für den Strahler 10 und für die Schutzgaszuführung integriert, die durch Verbindung des Grundkörpers 1a mit dem Anschlusskörper 1b betriebsbereit werden. Der Grundkörper 1a des Kopfteils 1 lässt sich werkzeuglos an dem quasi als Stecker ausgebildeten Anschlusskörper 1b befestigen, wenn etwa Rändelmuttern zur Befestigung an den Stehbolzen 95, die in dafür vorgesehenen Durchtrittsöffnungen den Grundkörper 1a durchdringen, verwendet werden. Durch die Anordnung der Stehbolzen 95 kann eine eindeutige und exakte Positionierung des Grundkörpers 1a gegenüber dem Anschlusskörper 1b sichergestellt werden, so dass die durch die Stehbolzen 95 gebildete genaue Führung einen gleichzeitigen und exakten Anschluss der Elemente für den Hochspannungsanschluss für den Strahler 10 und für die Schutzgaszuführung in dem Grund- und dem Anschlusskörper 1a, 1b bereitstellt. Insgesamt wird durch die erfindungsgemäß verbesserte Strahlereinheit mit Halterung und Kopfteil, die sowohl eine Schutzgaszufuhr als auch eine Längenanpassung der Halterung mit elektrischem Anschluss des Strahlers ermöglicht, eine einfache und sichere Montage und sowohl horizontal als auch vertikal exakte Positionierbarkeit des Strahlers in einem Hüllrohr sichergestellt.

Anstelle von Stehbolzen sind selbstverständlich auch andere Befestigungs- und/oder Führungsmittel, wie Schrauben, Stifte und/oder Schienen etc. denkbar, die eine lösbare Verbindung und exakte geführte Positionierung des Grundkörpers 1a an dem Anschlusskörper 1b gestatten. Eine lösbare Befestigung kann alternativ zu der auf das Gewinde des Stehbolzens aufgeschraubten Rändelmutter auch eine normale Mutter sein, oder es können andere Befestigungslösungen, etwa Splinte, die in eine entsprechende Öffnung quer zur Längserstreckung des Stifts eingeschoben werden, oder Klemm- oder Rastmechanismen eingesetzt werden.

Als Material für das Kopfteil kann ein temperaturbeständiger, gegebenenfalls auch UVstabiler Kunststoff, beispielsweise ein Fluorpolymer wie Polyvinylidenfluorid (PVDF) oder Polytetrafluorethylen (PTFE), gewählt werden, das zudem die elektrische Durchschlagsfestigkeit sicherstellt, so dass das Kopfteil berührungssicher ist. Wird PTFE als Material gewählt, so können zur Verbesserung der mechanischen Eigenschaften Füllstoffe, z. B. aus Glas, enthalten sein.

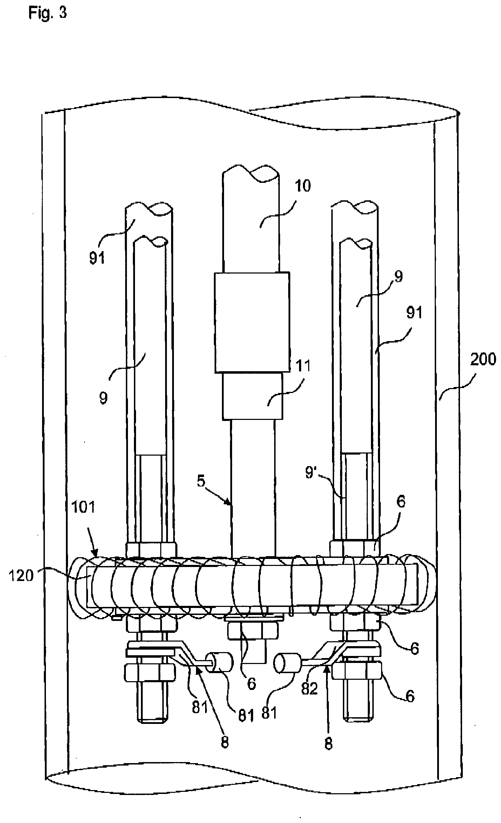

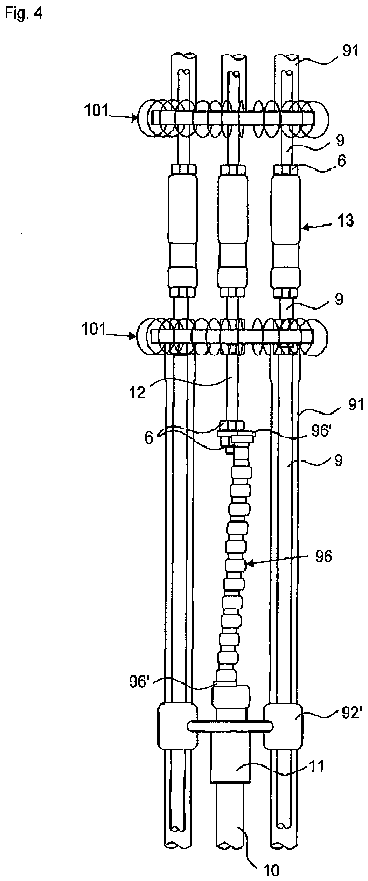

Weiter ist in **Fig. 1 und 2** zu sehen, dass sich von dem Kopfteil 1 das Gestänge 90 zur Halterung des Strahlers 10 erstreckt. In den mit den Figuren gezeigten Beispielen weist das Gestänge 90 drei Rohranordnungen auf, entsprechend der Anzahl der für den Anschluss des Strahlers 10 erforderlichen elektrischen Leitungen, die durch die Rohre 9 (vgl. **Fig. 3** und **4**) bereitgestellt werden. Die Rohre 9, die im Kopfteil 1 mit entsprechenden Hochspannungs- und Hochstromkontakten für den Hochspannungsanschluss verbunden sind, stellen somit den elektrischen Anschluss für den Strahler 10 bereit. Daher sind die Rohre 9 des Gestänges 90 von Isolatorrohren 91 umgeben, um Überschläge zu vermeiden.

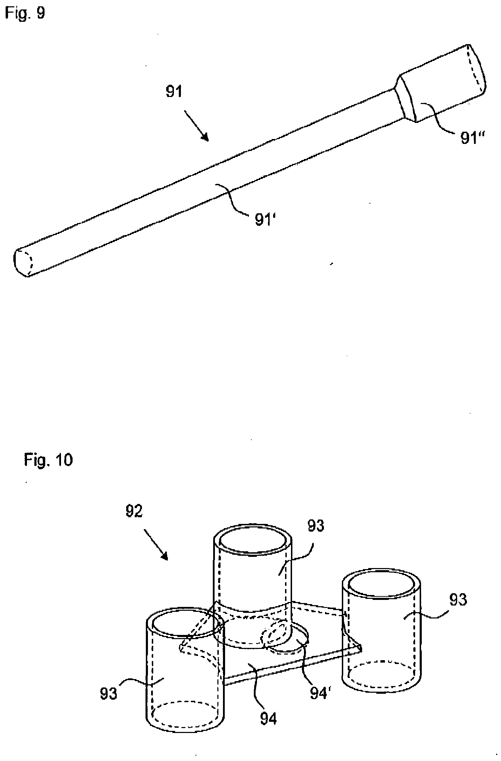

Ein Beispiel eines Isolatorrohrs 91 ist in **Fig. 9** dargestellt und besteht aus einem langen Rohrabschnitt 91' dessen Innendurchmesser ausreichend zur Aufnahme eines der Rohre 9 ist. An einem Ende weist das Isolatorrohr 91 einen aufgeweiteten Hülsenabschnitt 91" auf, dessen Innendurchmesser an den Außendurchmesser des Rohrabschnitts 91' angepasst ist, so dass dieser an seinem unaufgeweiteten Ende in dem Hülsenabschnitt 91" aufgenommen werden kann. Auf diese Weise kann die Länge des Isolators durch aneinander Reihung und ineinander Stecken mehrerer Isolatorrohre 9 angepasst werden, die auch unterschiedliche Längen aufweisen können, um die durch die Rohranordnungen vorgegebene Länge des Gestänges 90 zu erreichen.

Ähnlich kann die Länge jeder Rohranordnung durch eine Reihe miteinander verbundener Rohre 9 eingestellt werden, die hierzu beispielsweise an einem Ende wie in den Figuren gezeigt einen Außengewindeabschnitt 9' und an dem anderen Ende einen korrespondierenden Innengewindeabschnitt aufweisen können. Die Rohre 9, die ebenfalls unterschiedliche Längen zum Einstellen einer gewünschten Länge des Gestänges 90 aufweisen können, können so einfach miteinander verschraubt werden.

**Fig. 3** zeigt den unteren Strahleranschluss an dem kopfteilfernsten Zentrierelement 101 und **Fig. 4** den oberen Strahleranschluss, wobei ebenfalls Zentrierelemente 101 verwendet werden. Die vorliegende beispielhafte Strahlereinheit 100 weist somit drei Zentrierelemente 101 auf - allerdings können Anzahl und Anordnung der Zentrierelemente 101, etwa in Abhängigkeit der Gestängelänge, variieren. Eine der drei Rohranordnungen, die den wechselstromführenden Phasenleiter bildet, endet oberhalb des Strahlers 10, wobei der Anschluss des Strahlers 10 über eine elektrische Anschlussleitung 96 an dem oberen Strahlersockel 11 erfolgt. Die beiden weiteren Rohranordnungen (Neutralleiter zum Potentialausgleich) erstrecken sich bis unterhalb des Strahlers 10 und werden elektrisch mit dem Strahler 10 durch ein Zentrierelement 101 (auch als kopfteilfernstes Zentrierelement bezeichnet) verbunden. Oberhalb des Strahlers 10 sind Zentrierelemente 101 oberhalb und unterhalb von Verbindungshülsen 13 angeordnet, die der mechanischen und elektrischen Verbindung zweier Innengewindeabschnitte von Rohren 9 dienen bzw. der Verbindung eines der Rohre 9 mit einem Anschlussrohr 12, das mit der Anschlussleitung 96 verbunden ist.
Um die Rohre 9 des Gestänges 90 in ihrer parallelen Anordnung über die gesamte Länge des Gestänges 90 stabil zu halten, sind Isoliersterne 92 vorgesehen, die jeweils oberhalb der Steckverbindung ineinander gesteckter Isolatorrohre 91 angeordnet sind. Ein solcher Isolierstern 92 ist in **Fig. 10** dargestellt. Der Isolierstern 92, der wie die Isolatorrohre 91 beispielsweise aus Glas, insbesondere Quarzglas gefertigt sein kann, weist hier drei parallele Hülsen 93 auf, in denen die drei Rohre 9 bzw. die diese umgebenden Isolatorrohre 91 geführt werden. Hierzu weisen die Hülsen 93 einen entsprechend passenden Durchmesser auf. Die Hülsen 93 sind über ein Verbindungsstück 94 verbunden und dadurch in ihrer Position zueinander festgelegt. Das Verbindungsstück 94 weist eine Öffnung 94' auf, die in der Anordnung am Gestänge 90 axial-zentriert liegt. Diese Öffnung 94' dient im Zentrierstern 92', der einem Isolierstern 92 entsprechen kann, der zentrierten Halterung des oberen Strahlersockels 11, wie in **Fig. 4** zu sehen ist. Für den Zentrierstern 92' sind allerdings entsprechend der dort vorliegenden Anzahl an Rohren 9 hier lediglich zwei Hülsen 93 ausreichend. Die Anzahl der Hülsen 93 am Isolierstern/Zentrierstern 92, 92' kann somit der Anzahl der zu führenden Rohre angepasst sein - es kann aber auch vorgesehen sein, einen Isolierstern 92 mit drei Hülsen 93 als Zentrierstern 92' einzusetzen, wobei eine der Hülsen 93 ungenutzt bleibt. Falls das Gestänge mehr als drei Rohre/Stangen aufweist, kann ein Isolier- bzw. Zentrierstern auch entsprechend mehr Hülsen aufweisen, die in der für das jeweilige Gestänge vorgesehenen Anordnung über ein Verbindungsstück verbunden sind.

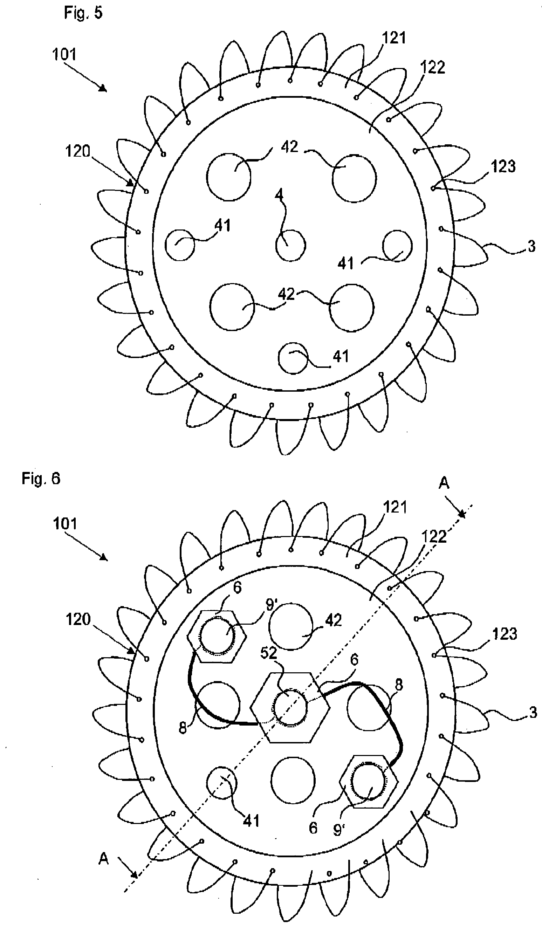

**Fig. 5** zeigt eine Ausführungsform eines Zentrierelements 101. Der kreisrunde Scheibengrundkörper 120 weist in einem Zentralabschnitt 122 eine axial-zentrale Bohrung 4 auf, die, wenn das Zentrierelement 101 als kopfteilfernstes Zentrierelement eingesetzt wird, der Befestigung des hier nicht dargestellten Strahlers 10 dient. Im abgesetzten Randabschnitt 121, dessen Dicke hier geringer ist als die des Zentralabschnitts 122, aber dies nicht zwingend sein muss, weist der Scheibengrundkörper 120 eine Vielzahl von axialparallelen Durchtrittsbohrungen 123 auf, durch die ein Federdraht 3 als elastisches Federelement gewunden ist. Die Dicke des Randabschnitts 121 ist so gewählt, dass die Windungen der Drahtwicklung 3 beim Durchtritt durch die Öffnungen 123 nicht aufgebogen werden. Sollte die Dicke des Scheibenkörpers für die gewählte Drahtwicklung ausreichend gering sein, so ist, anders als in den Figuren dargestellt, ein abgesetzter Randabschnitt nicht erforderlich.

Die axial-zentrale Bohrung 4 kann eine einfache zylindrische Durchtrittöffnung ohne Gewinde oder Formschlusselemente sein, sodass ein in die Bohrung eingeführtes Anschlusselement befestigt werden kann. Zum lösbaren Befestigen bieten sich Schraube-Mutter-Verbindungen an. Es sind aber auch Steck- oder Umformlösungen denkbar.

Entsprechendes gilt für die weiteren Bohrungen 41 in dem Scheibengrundkörper 2, die in **Fig. 5** dargestellt sind. Die weiteren Bohrungen 41 - im vorliegenden Fall drei, es können aber auch weniger oder mehr sein - sind zur direkten Aufnahme jeweils eines Rohres 9 des Gestänges 90 vorgesehen (vgl. **Fig. 3** **und** **4**). Es ist aber auch denkbar, dass die weiteren Bohrungen 41 ein Anschlusselement für das Gestänge aufweisen können. Die Ausnehmungen 42, die Durchgangsbohrungen oder auch nur dickenreduzierte Bereiche sein können, dienen der Gewichtsreduktion des Scheibengrundkörpers 2, der vorzugsweise aus einer Keramik gefertigt ist. Ferner kann der Scheibengrundkörper 101 auch aus einem Kunststoffmaterial, insbesondere einem temperatur- und wärmestabilen Hochleistungskunststoff bestehen. Gegebenenfalls kann der Scheibengrundkörper 101 auch aus Metall, etwa Edelstahl gefertigt sein, wobei allerdings eine elektrische Isolierung der in den Bohrungen 41 aufgenommenen Rohre 9 vorzusehen ist. Sind die gewichtsreduzierenden Ausnehmungen 42 Durchgangsbohrungen, kann hier zusätzlich zu dem umfänglichen Spalt, der durch das Federelement 3 zwischen einem Hüllrohr 200 und dem Zentrierelement 101 bleibt, auch ein Durchtritt von Schutzgas erfolgen, das aus den offenen Rohrenden der Rohre 9 unterhalb des kopfteilfernsten Zentrierelements 101 (vgl. **Fig. 3**) bzw. des Anschlussrohrs 12, das durch die Verbindungshülse 13 mit einem der Rohre 9 verbunden ist, unterhalb des kopfteilnäheren Zentrierelements 101 (vgl. **Fig. 4**) austreten kann.

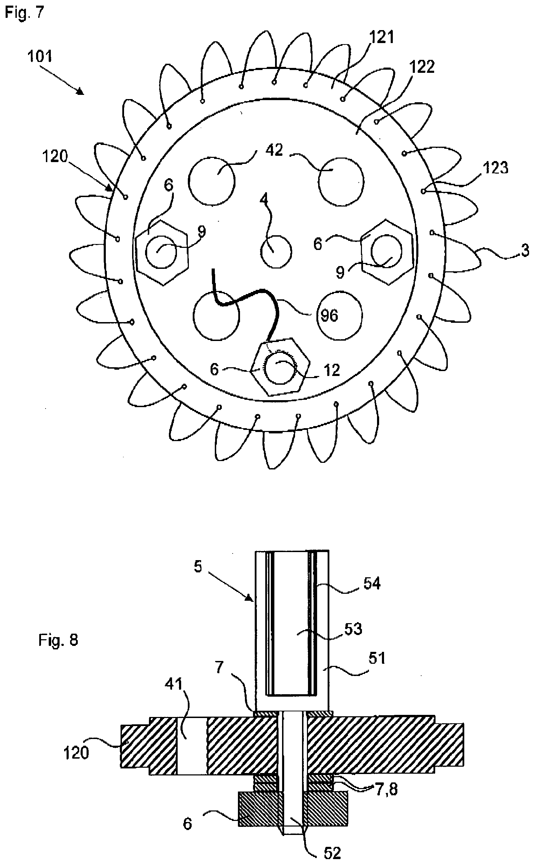

**Fig. 6** zeigt in vereinfachter Weise die elektrische Kontaktierung des Strahlers 10 am kopfteilfernsten Zentrierelement 101 mittels Kontaktierungselementen 8 zwischen den Gewindeabschnitten 9' der beiden Rohre 9 (vgl. **Fig. 3**) in zwei gegenüberliegenden exzentrischen Bohrungen 41 des Zentrierelements 101 und einem Befestigungsabschnitt 52 eines Anschlusselements 5 (vgl. **Fig. 8**) in der axial-zentralen Bohrung 4, das zum Anschluss des unteren Strahlersockels 11 (vgl. **Fig. 3**) dient.

Das Anschlusselement 5 weist einen hülsenartigen Anschlussabschnitt 51 und einen zapfenartigen Befestigungsabschnitt 52 auf. Die Aufnahmeöffnung 53 im hülsenartigen Anschlussabschnitt 51 weist zur lösbaren elektrischen Kontaktierung und zur lösbaren mechanischen Befestigung des Lampensockels 11 (vgl. **Fig. 3**) an ihrer Innenmantelfläche Befestigungs- und Kontaktierungsmittel 53 wie beispielsweise Gewinde, Federdrähte oder Lamellenbleche auf. Bevorzugt können als Anschlusselemente 5 Kontaktstecker ODU Springtac® oder ODU Lamtac® der Steckverbindungssysteme der ODU GmbH & Co. KG, Mühldorf am Inn, DE, eingesetzt werden. Der Lampensockel 11 wird mit einem entsprechenden Gegenstück, das in dem hülsenartigen Anschlussabschnitt 51 aufgenommen werden kann, ausgestattet. Generell ist auch denkbar, dass ein Anschlussabschnitt eines Anschlusselements zapfenartig ausgebildet ist, so dass der Lampensockel bzw. das Gestänge mit einer entsprechenden Hülse ausgebildet oder ausgestattet sein wird.

In den vorliegenden Beispielen weist der Befestigungsabschnitt 52, der sich durch die axial-zentrale Bohrung 4 bzw. die exzentrischen Bohrungen 41 erstreckt, ein Außengewinde auf, so dass das Anschlusselement 5 an dem Scheibengrundkörper 2 mittels einer Schraubmutter 6 gesichert werden kann. Üblicherweise kommt zwischen dem Scheibengrundkörper 2 und der Schraubmutter 6 bzw. dem Anschlussabschnitt 51 eine Unterlegscheibe 7 zu liegen. Ferner wird durch die Schraubmutter 6 ein elektrisches Kontaktelement 8 mit dem Anschlusselement 5 verbunden.

Der Gewindeabschnitt 9' des Rohrs 9 dient nicht nur zur Verbindung zweier Rohre, sondern kann auch zur Befestigung am Zentrierelement 101 verwendet werden. Dabei erstreckt sich der Gewindeabschnitt 9' durch eine der exzentrischen Bohrungen 41. Beidseitig des Zentrierelements 1 sind Muttern 6 zur Fixierung des Rohres 9 gezeigt.

Das elektrische Kontaktelement 8 kann, wie in **Fig. 3** angedeutet ist, eine abgewinkelte Metalllasche 82 mit einer Aderendhülse 81 aufweisen. Die Metalllasche 82 umgibt kontaktierend den Gewindeabschnitt 9' des jeweiligen Rohres 9 und wird mit einer weiteren Mutter 6 befestigt. In der Aderendhülse 81 kann ein Ende einer Drahtlitze (nicht dargestellt) aufgenommen und durch Crimpen der Aderendhülse 81 befestigt werden. Auch das andere Ende der Drahtlitze kann in einer entsprechenden Aderendhülse 81 aufgenommen sein, die über eine Metalllasche in entsprechender Weise an dem Befestigungsabschnitt 52 des Anschlusselements 5 für den Strahler 10 befestigt werden, so dass ein elektrischer Kontakt zwischen dem Gestänge 9 und dem Strahler 10 hergestellt wird. Alternativ zur Litze können auch Metallstreifen zur Kontaktierung verwendet werden. So wird der elektrische Anschluss des Strahlers 10 über die Anschlusselemente 5 und die Rohre 9 des Gestänges 90 hergestellt, das zudem der Halterung des Strahlers 10 in dem Hüllrohr 200 dient.

Auch die nicht dargestellte Litze, die den elektrischen Kontakt herstellt, wird durch einen Isolator umhüllt, hierfür kommen z. B. kurze Glas- oder Keramikhülsen in Fragen. Je nach Strahlertyp, d. h. Betriebstemperatur und Emissionsspektrum, kann gegebenenfalls zur Isolierung auch ein temperatur- (und UV-)stabiler Hochleistungskunststoff verwendet werden. Selbstverständlich kann die elektrische Kontaktierung zwischen den Anschlusselementen auch auf eine von der hier beispielhaft dargelegten Weise abweichende Variante hergestellt werden. Dem Fachmann sind hierzu elektrische Kontaktierungsmittel bekannt.

Weiter ist in **Fig. 3** zu sehen, wie das Zentrierelement 101 durch das etwas radial komprimierte elastische Federelement 3 im Hüllrohr 200 anliegt und so für die zentrierte Anordnung des Strahlers 10 sorgt. Der Scheibengrundkörper 120, dessen Durchmesser kleiner ist als der Innendurchmesser des Hüllrohrs 200, ist umfänglich durch das elastische Federelement 3 gleichmäßig von der Innenwandung des Hüllrohrs 200 entfernt.

Der weitere Anschluss des Strahlers 10 am oberen Sockel 11 ist in **Fig. 4** und **Fig. 7** zu sehen. Das Anschlussrohr 12, das mit dem Rohr 9, das den stromführenden Leiter bildet, mittels der Verbindungshülse 13 verbunden ist und unterhalb des Zentrierelements 101 endet, das sich oberhalb des Strahlers 10 befindet, steht in elektrisch leitendem Kontakt mit einer Anschlussleitung 96, die an dem oberen Strahlersockel 11 angeschlossen ist. Die elektrische Isolierung der Anschlussleitung 96 kann auch hier in Abhängigkeit des Strahlertyps bzw. dessen Betriebstemperatur und Emissionsspektrum durch eine Reihe von Glas- oder Keramikhülsen oder durch einen Hochleistungskunststoff bereitgestellt werden. Im gezeigten Beispiel ist die Anschlussleitung 96 über eine Metalllasche 96' an dem Gewindeabschnitt des Anschlussrohrs 12 befestigt, um den radialen Versatz zwischen dem exzentrisch angeordneten Anschlussrohr 12, das eines der Rohre 9 verlängert, und dem axial-zentriert angeordneten Strahlersockel 11 des Strahlers 10 zu überbrücken. Ähnlich ist die Anschlussleitung 96 mit dem oberen Strahlersockel 11 über eine Lasche 96' verbunden. Alternative Anschlüsse ohne Laschen oder mit anderen Brückenelementen sind aber ohne weiteres denkbar. Die Anschlussleitung 96 kann zudem länger sein als der Abstand zwischen der Befestigung an dem Anschlussrohr 12 und dem oberen Strahlersockel 11. Auf diese Weise können thermische Ausdehnungseffekte kompensiert werden.

Dies wird unterstützt durch das Zentrierelement 101, das axiale Verschiebung zulässt, so dass keine Spannungen in dem Gestänge 9, dem Strahler 10 und dem Hüllrohr 200 auftreten.

Neben dem elektrischen Anschluss sorgen die Rohre 9 des Gestänges 90, die im Kopfteil 1 auch mit den Elementen für die Schutzgaszuführung verbunden sind, für eine redundante Zuführung von Schutzgas an die Strahleranschlüsse bzw. den erforderlichen Stellen im System.

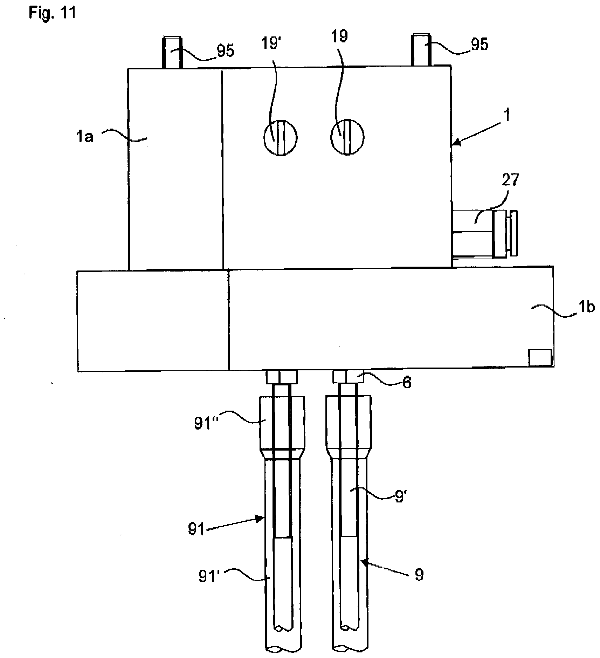

**Figuren 11 bis 17** zeigen das Kopfteil 1 und seine Teile Grundkörper 1a und Anschlusskörper 1b im Detail mit den Elementen zum Anschluss der Rohre 9 des Gestänges zur elektrischen Kontaktierung und zur Schutzgaszufuhr.

In der Seitenansicht in **Fig. 11** sind zwei Rohre 9 zu sehen, die mit dem Anschlusskörper 1b verbunden sind. Das dritte, für den elektrischen Anschluss des Strahlers 10 erforderliche Rohr ist hier verdeckt hinter dem rechten Rohr 9. Die Rohre 9 sind von Isolatorrohren 91 umgeben, deren aufgeweitete Hülse 91" nach oben zum Anschlusskörper 1b weist. Gegebenenfalls kann die Mutter 6, mit der das Rohr 9 an dem Anschlusskörper 1b befestigt ist, auch in der Hülse 91" aufgenommen werden, wenn das Isolatorrohr 91 weiter nach oben bis zum Anschlag an den Anschlusskörper 1b geschoben wird. Weiter sind die freien Enden der Stehbolzen 95 zu sehen, die über den Grundkörper 1a hinausragen, um diesen - beispielsweise durch Aufschrauben (nicht gezeigter) Rändelmuttern - am Anschlusskörper 1b festzulegen. Diese Befestigung kann einfach wieder durch Abschrauben der Muttern gelöst werden. Sowohl Befestigung als auch Lösen kann vorteilhaft werkzeuglos händisch erfolgen. Zu sehen sind ferner zwei Verbindungsbohrungen 19, 19' an einer Seite des Grundkörpers 1a sowie eine Gasanschlussbuchse 27 an der benachbarten Seite des Grundkörpers 1a.

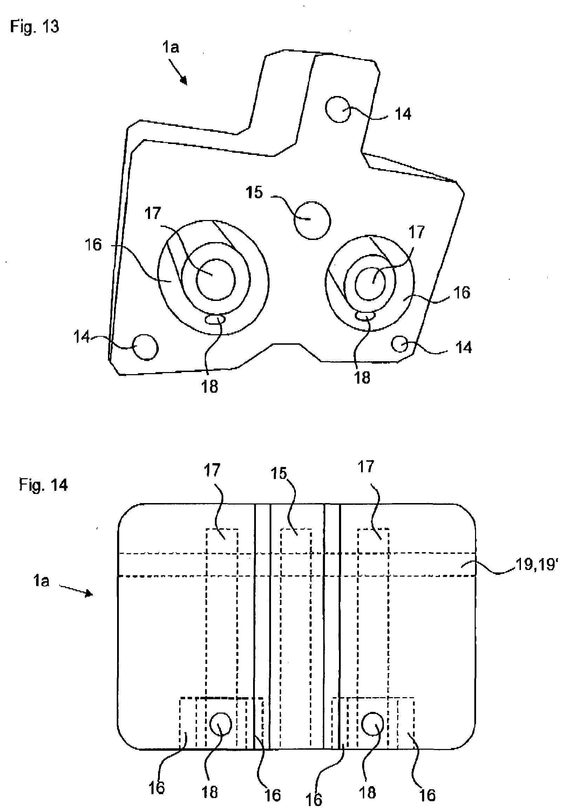

Der Aufbau eines beispielhaften Grundkörpers 1a ist in **Fig. 13, 14** **und** **15** dargestellt. Der Grundkörper 1a hat hier eine polygonale, in etwa T-förmige Querschnittform und weist eine Vielzahl von Bohrungen auf. Allerdings können Grundkörper und Anschlusskörper eines Kopfteils auch von der dargestellten Form abweichende Querschnitte haben. Die dargestellte Form verbindet vorteilhaft ein möglichst geringes Gewicht und eine gute Handhabbarkeit und unterstützt den korrekten Zusammenbau von Anschluss- und Grundkörper.

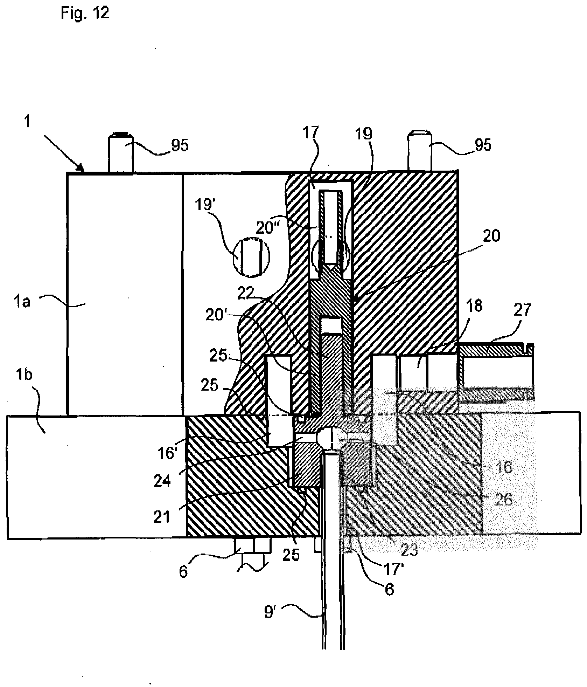

Die Bohrungen 14 (nur in **Fig. 13** dargestellt) sind Durchtrittsbohrungen und dienen der Aufnahme der Stehbolzen 95 zur Befestigung am Anschlusskörper 1b. Die Bohrungen 15 und 17 sind im vorliegenden Beispiel Sackbohrungen und sind zur Aufnahme von Elementen für den elektrischen Anschluss der Rohre 9 vorgesehen. Dabei kann es sich, wie in **Fig. 12** beispielhaft dargestellt, um eine dem Anschlusselement 5 entsprechende Steckhülse 20 handeln, die als Teil der Steckverbindungssysteme der ODU GmbH & Co. KG, Mühldorf am Inn, DE, erhältlich sind. Die Steckhülse 20 weist einen Hülsenabschnitt 20' und einen Kontaktabschnitt 20" auf und ist derart in der Bohrung 15 bzw. 17 angeordnet, dass das offene Ende des Hülsenabschnitts 20' zum Anschlusskörper 1b weist und sich der Kontaktabschnitt 20" bis zu der Verbindungsbohrung 19,19' erstreckt. Der Durchmesser der Bohrung 15,17 ist an den Außendurchmesser des Hülsenabschnitts 20' angepasst, so dass die Steckhülse 20 gegebenenfalls mit etwas Spiel in der Bohrung 15,17 aufgenommen wird.

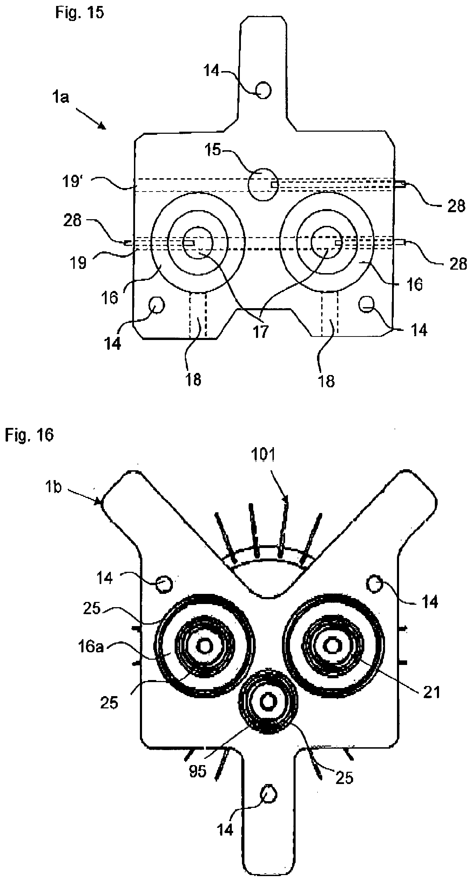

Durch die Verbindungsbohrungen 19,19' können elektrische Kontaktelemente 28 (vgl. Fig. 15) von außen eingeführt werden, um mit dem jeweiligen Kontaktabschnitt 20" in Kontakt gebracht zu werden.

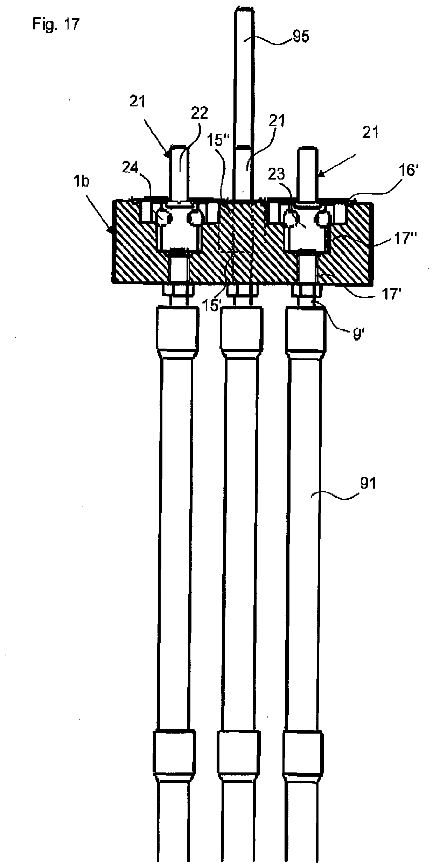

Die elektrische Verbindung von der Steckhülse 20, die im Grundkörper 1a des Kopfteils 1 angeordnet ist, zu dem jeweiligen Rohr 9 erfolgt über ein weiteres Kontaktelement, hier Verbindungsstecker 21, der in **Fig. 12** **und** **17** zu sehen ist. Der Anschlusskörper 1b weist für die Verbindungsstecker 21 auf der dem Grundkörper 1a zugewandten Seite an jeder Durchtrittsbohrung 15', 17' eine abgestuft aufgeweitete Öffnung 15", 17" auf. Die Durchtrittsbohrungen 15', 17' im Anschlusskörper 1 b, in denen jeweils ein Rohr 9 mit seinem Ende am Gewindeabschnitt 9' aufgenommen ist, fluchten mit den Bohrungen 15,17 im Grundkörper 1a, und der Durchmesser der abgestuft aufgeweiteten Öffnung 15", 17" ist zur Aufnahme eines Aufnahmeabschnitts 23 des Verbindungssteckers 21 ausgebildet. Der Aufnahmeabschnitt 23 weist vorliegend ein Innengewinde korrespondierend zum Außengewindeabschnitt 9' des Rohrs 9 auf, so dass das Rohr 9 mit Aufnahmeabschnitt 23 des Verbindungssteckers 21 verschraubt werden kann. Von dem Aufnahmeabschnitt 23 des Verbindungssteckers 21 erstreckt sich ein Zapfenabschnitt 22 über den Anschlusskörper 1b hinaus in Richtung des Grundkörpers 1a, wobei der Zapfenabschnitt 22 geformt und dimensioniert ist, um in den Hülsenabschnitt 20' der Steckhülse 20 aufgenommen zu werden. Der elektrische Anschluss des Strahlers 10 erfolgt also über die elektrischen Kontaktelemente 28 in den Verbindungsbohrungen 19,19', über die Steckhülse 20 in der Bohrung 15,17 im Grundkörper 1a und über den Verbindungsstecker 21 in der abgestuft aufgeweiteten Öffnung 15", 17" der Durchtrittsbohrung 15', 17' im Anschlusskörper 1b zu dem jeweiligen Rohr 9. Sind mehr als drei elektrische Anschlussleitungen erforderlich, kann das Kopfteil 1 entsprechend mehr Bohrungen und Verbindungsbohrungen mit den jeweiligen elektrischen Anschlusselementen aufweisen.

Zur Schutzgaszufuhr weist der Grundkörper 1a des Kopfteils 1 konzentrische Sackringbohrungen 16 um die Bohrungen 17 auf, wobei in jede Sackringbohrung 16 jeweils zumindest eine Verbindungsbohrung 18 mündet. In dem mit den Figuren gezeigten Beispiel erstrecken sich die Verbindungsbohrungen 18 rechtwinklig in Bezug zu den Verbindungsbohrungen 19,19' durch den Grundkörper 1a, wobei die Verbindungsbohrungen 19,19' beabstandet zu den Sackringbohrungen 16 verlaufen. Anders als dargestellt, kann auch nur eine Sackringbohrung um eine der Bohrungen 17 oder auch drei Sackringbohrungen um jede der Bohrungen 15,17 zur Schutzgaszufuhr vorgesehen sein. Weist das Gestänge mehr als drei Rohranordnungen auf, so können auch entsprechend mehr Schutzgaszufuhrelemente im Kopfteil vorgesehen sein.

Die Durchtrittsöffnungen 17' im Anschlusskörper 1b weisen eine weitere abgestuft aufgeweitete Öffnung 16' auf, die im Durchmesser mit den Sackringbohrungen 16 im Grundkörper 1a korrespondieren und die zusammen mit der Sackringbohrung 16 im zusammengebauten Zustand des Kopfteils 1 einen Schutzgaszufuhrraum bilden, dem durch die Verbindungsbohrung 18, an die sich eine Anschlussbuchse 26 zum Anschluss einer (nicht dargestellten) Schutzgasleitung anschließen kann, Schutzgas zugeführt werden kann. Der Schutzgaszufuhrraum kann durch ringförmige Dichtungen, die in umlaufenden Ringnuten 25 an Kontaktflächen des Anschlusskörpers 1b und des Verbindungssteckers 21 zum Grundkörper 1a angeordnet sind, abgedichtet werden. Dichtungen können an der Stirnfläche des Anschlusskörpers 1b um die abgestuft aufgeweitete Öffnung 16' und auch 15", um die Durchtrittsöffnung 17' an der ersten aufgeweiteten Stufe 17" und auf dem Aufnahmeabschnitt 23 des Verbindungssteckers 21 vorgesehen sein. Letztere kommt dichtend an dem Ringsteg zur Anlage, der zwischen der Sackringbohrung 16 und der Bohrung 17 vorliegt.

Aus dem durch die Sackringbohrung 16 und die aufgeweitete Öffnung 16' gebildeten Schutzgaszufuhrraum gelangt das Schutzgas über Verbindungsbohrungen 24, die sich durch den Aufnahmeabschnitt 23 in einen Hohlraum 26 oberhalb des Innengewindes erstrecken, in das dort eingefügte Rohr 9.

In den vorliegenden Beispielen wird beschrieben, dass die Rohre durch Gewinde miteinander verbunden und an Zentrierelement und Anschlusskörper befestigt sind. Es ist aber selbstverständlich, dass auch davon abweichende Verbindungslösungen vorgesehen sein können, wie etwa Bajonett-Verschluss oder Stecklösungen, wobei die Befestigungsmechanismen im Zentrierelement und im Anschlusskörper in äquivalenter Weise von Schraubverbindungen abweichend ausgebildet sein können.

Durch die Schutzgaszufuhr kann um den Strahler 10 in dem Hüllrohr 200 eine Schutzgasatmosphäre bereitgestellt werden, die auch einen Überdruck gegenüber der Umgebung um das Hüllrohr aufweisen kann, so dass selbst im Fall eines Defekts des Hüllrohrs zum einen durch den Überdruck ein Eindringen potentiell explosiver Stoffe verhindert oder zumindest verringert wird, zum anderen durch das Fehlen von Sauerstoff ein Zünden an dem Strahler eventuell eingetretener Stoffe verhindert wird. Zur Bereitstellung und Aufrechterhaltung der Schutzgasatmosphäre können Vorrichtungen und Elemente zur Abdichtung des Hüll- bzw. Tauchrohrs an dem Kopfteil vorgesehen sein und ferner Ventile und/oder Drucksensoren etc., mit denen ein vorgesehener Schutzgasdruck eingestellt und gegebenenfalls überwacht werden kann.

Weiter kann zusätzlich zu den Elementen zur Schutzgaszufuhr auch eine Schutzgasabfuhr vorgesehen sein, wenn eine Schutzgasspülung vorgesehen ist. In einfacher Weise könnte dazu einfach eine Lücke zwischen Hüll- bzw. Tauchrohr und Kopfteil vorgesehen sein, aus der das Schutzgas entweichen kann. Das Kopfteil kann aber auch Elemente aufweisen, die eine gesteuerte bzw. geregelte Schutzgasabfuhr ermöglichen. Hierzu sind Ventile in oder an zumindest einer zusätzlichen Bohrung durch das Kopfteil denkbar, die sich von der strahlernahen Seite des Anschlusskörpers durch diesen und durch eine entsprechend korrespondierende Bohrung im Grundkörper erstreckt.

**BEZUGSZEICHENLISTE**

| | | | |
|---|---|---|---|
| 1 | Kopfteil | 200 | Hüllrohr |
| 1a, 1b | Grund-, Anschlusskörper | | |
| 10 | Strahler | 3 | Federelement, Drahtwicklung |
| 11 | Strahlersockel | 4 | axial-zentrale Bohrung |
| 12 | Anschlussrohr | 41 | exzentrische Bohrung für Gestänge |
| 13 | Verbindungshülse | 42 | Ausnehmungen |
| 14 | Durchtrittsöffnung | | |
| 14' | Durchtritts- oder Sackbohrung | 5 | Anschlusselement |
| 15 | Sackbohrung | 51 | Anschlussabschnitt/Hülse |
| 15', 15" | Durchtritts-, abgestufte Öffnung in 1b | 52 | Befestigungsabschnitt/Zapfen |
| 16 | Sackringbohrung | 53 | Aufnahmeöffnung |
| 16' | abgestufte Öffnungen in 1b | 54 | Kontaktierungsmittel |
| 17 | Sackbohrung | | |
| 17',17" | Durchtritts-, abgestufte Öffnung in 1b | 6 | Mutter |
| 18 | Verbindungsbohrung zu 16 | 7 | Unterlegscheibe |
| 19,19' | Verbindungsbohrung zu 17,15 | 8 | Kontaktelement |
| 20 | Steckhülse | 81 | Aderendhülse |
| 20', 20" | Hülsenabschnitt, Kontaktabschnitt | 82 | Metalllasche |
| 21 | Verbindungsstecker | 90 | Gestänge |
| 22 | Zapfenabschnitt | 9 | Rohr |
| 23 | Aufnahmeabschnitt | 9' | Außengewindeabschnitt |
| 24 | Verbindungsbohrung | 91 | Isolatorrohr |
| 25 | Nut für Dichtring | 91' | Rohrabschnitt |
| 26 | Hohlraum | 91" | Hülsenabschnitt |
| 27 | Anschlussbuchse | 92 | Isolierstern |
| 28 | elektrisches Kontaktelement | 92' | Zentrierstern |
| 100 | Strahlerhalterung | 93 | Hülse |
| 101 | Zentrierelement | 94 | Verbindungsstück |
| 120 | Scheibengrundkörper | 94' | Öffnung |
| 121 | abgesetzter Randabschnitt | 95 | Stehbolzen |
| 122 | Zentralabschnitt | 96 | el. Anschlussleitung, isoliert |
| 123 | Durchtrittöffnungen | 96' | Anschlusslasche |

## Patentansprüche

1. Strahlereinheit (100) mit Kopfteil (1) und Halterung für einen Strahler (10), wobei das Kopfteil (1) einen strahlerfernen Grundkörper (1a) aus einem elektrisch isolierenden Material aufweist, der eine elektrische Anschlusseinrichtung und eine Schutzgaszufuhreinrichtung aufweist, und wobei
die Strahlereinheit (100) ein Gestänge (90) als Halterung für den Strahler (10) aufweist, das aus einer Mehrzahl Rohre (9) oder aus einer Mehrzahl Stangen und zumindest einem Rohr (9) aus einem elektrisch leitenden Material besteht,
wobei die für den Strahler (10) erforderlichen elektrischen Anschlussleitungen durch zumindest einen Teil der Rohre (9) oder Stangen gebildet werden, wobei die jeweiligen Rohre und/oder Stangen einenends mit der elektrischen Anschlusseinrichtung in Kontakt stehen und an ihrem anderen Ende den elektrischen Anschluss des Strahlers (10) bereitstellen,
und wobei zumindest eines der Rohre (9) einenends mit der Schutzgaszufuhreinrichtung in fluidischer Kommunikation steht und anderenends die Schutzgaszufuhr zum Strahler (10) bereitstellt,
**dadurch gekennzeichnet, dass**
das Kopfteil (1) einen strahlernahen Anschlusskörper (1b) aus einem elektrisch isolierenden Material aufweist, der lösbar mit dem Grundkörper (1a) verbunden ist und der parallel orientierte Durchtrittsöffnungen (15', 17') für die Rohre (9) oder für die Stangen und das Rohr (9) aufweist, die an dem Anschlusskörper (1b) lösbar befestigt sind, und
der Grundkörper (1a) Bohrungen (15,17) aufweist, die mit den Durchtrittsöffnungen (15', 17') im Anschlusskörper (1b) fluchten,
wobei der elektrische Kontakt der jeweiligen Rohre (9) und/oder Stangen, die die elektrischen Anschlussleitungen bilden, mit der elektrischen Anschlusseinrichtung und die fluidische Kommunikation des zumindest einen Rohrs (9), das zur Schutzgaszufuhr zum Strahler (10) vorgesehen ist, mit der Schutzgaszufuhreinrichtung durch die Verbindung des Grundkörpers (1a) mit dem Anschlusskörper (1b) bereitgestellt wird, wobei sich durch den Grundkörper (1a) Verbindungsbohrungen (19,19') von zumindest einer der Seiten des Grundkörpers (1a) zu den Bohrungen (15,17) in einem von dem Anschlusskörper (1b) entfernt liegenden Bereich erstrecken, wobei
in den Verbindungsbohrungen (19,19') für jede der für den Strahler (10) erforderlichen Anschlussleitungen jeweils ein elektrisches Kontaktelement (28) der elektrischen Anschlusseinrichtung und
in den Bohrungen (15,17) jeweils eine Steckhülse (20) vorliegen, die durch das elektrische Kontaktelement (28) kontaktiert wird und direkt oder über einen Verbindungsstecker (21) mit jeweils einem der Rohre (9), die in den Durchtrittsöffnungen (15', 17') des Anschlusskörpers (1b) aufgenommen sind, verbunden ist, und wobei zumindest eine Bohrung (17) der Bohrungen (15,17) im Grundkörper (1a) von einer Sackringbohrung (16) konzentrisch umgeben ist, deren Tiefe kleiner ist als ein Abstand zu den Verbindungsbohrungen (19,19') und in die jeweils eine weitere Verbindungsbohrung (18) von zumindest einer der Seiten des Grundkörpers (1a) mündet, wobei die weitere Verbindungsbohrung (18) und die Sackringbohrung (16) die Schutzgaszufuhreinrichtung bereitstellen.

2. Strahlereinheit (100) nach Anspruch 1,
**dadurch gekennzeichnet, dass**
die Steckhülse (20) einen Hülsenabschnitt (20'), dessen offenes Ende zum Anschlusskörper (1b) weist, und einen Kontaktabschnitt (20") aufweist, der sich zumindest bis zu der Verbindungsbohrung (19,19') erstreckt, wobei der Verbindungsstecker (21) einen Aufnahmeabschnitt (23) für eines der Rohre (9) und einen Zapfenabschnitt (22) aufweist, der in den Hülsenabschnitt (20') der Steckhülse (20) aufnehmbar oder aufgenommen ist.

3. Strahlereinheit (100) nach Anspruch 1 oder 2,
**dadurch gekennzeichnet, dass**
in dem Anschlusskörper (1b) sich die Durchtrittsöffnung (17') zum Grundkörper (1a) hin abgestuft aufweitet, wobei eine erste abgestufte Öffnung (17") zur Aufnahme des Aufnahmeabschnitts (23) des Verbindungssteckers (21) ausgebildet ist, wobei der Zapfenabschnitt (22) des Verbindungssteckers (21) über den Anschlusskörper (1b) hinausragt, und eine zweite abgestufte Öffnung (16') im Durchmesser dem Außendurchmesser der Sackringbohrung (16) im Grundkörper (1a) entspricht und damit fluchtet, wenn der Grundkörper (1a) und der Anschlusskörper (1b) verbunden sind.

4. Strahlereinheit (100) nach zumindest einem der Ansprüche 1 bis 3,
**dadurch gekennzeichnet, dass**
der Aufnahmeabschnitt (23) des Verbindungssteckers (21) radiale Verbindungsbohrungen (24) zu einem im Verbindungsstecker (21) vorliegenden Hohlraum (26) aufweist, der mit einer Mündung des Rohrs (9), das in dem Aufnahmeabschnitt (23) aufgenommen ist, verbunden ist.

5. Strahlereinheit (100) nach zumindest einem der Ansprüche 1 bis 4,
**dadurch gekennzeichnet, dass**
die lösbare Verbindung des Grundkörpers (1a) mit dem Anschlusskörper (1b) durch Stifte, Schrauben oder Stehbolzen (95) bereitgestellt wird, die in Öffnungen (14') des Anschlusskörpers (1b) festgelegt sind und eine Länge zur Durchdringung des Grundkörpers (1a) aufweisen, der mit den Öffnungen (14') fluchtende Durchtrittsöffnungen (14) aufweist.

6. Strahlereinheit (100) nach zumindest einem der Ansprüche 1 bis 5,
**dadurch gekennzeichnet, dass**
jedes Rohr (9) durch eine Rohranordnung aus mehreren aneinandergereihten Rohren (9) gebildet wird, die an den Enden miteinander korrespondierende Eingriffselemente, vorzugsweise einenends einen Außengewindeabschnitt (9') und anderenens einen korrespondierenden Innengewindeabschnitt, aufweisen, sodass eine gewünschte Länge der Halterung mit einer Rohranordnung durch Verbinden, vorzugsweise Verschrauben mehrerer Rohre (9) einstellbar ist.

7. Strahlereinheit (100) nach zumindest einem der Ansprüche 1 bis 6,
**dadurch gekennzeichnet, dass**
jedes Rohr (9) des Gestänges (90) von einem Isolatorrohr (91) umgeben ist, das bevorzugt einen Rohrabschnitt (91') und einen endständigen aufgeweiteten Hülsenabschnitt (91") aufweist, so dass in Abhängigkeit der gewünschten Länge der Halterung mehrere Isolatorrohre (91) zu einer Isolatorrohranordnung zusammen steckbar sind, wobei ein Endabschnitt des Rohrabschnitts (91') eines ersten Isolatorrohrs (91) in dem Hülsenabschnitt (91") eines zweiten Isolatorrohrs (91) aufgenommen wird.

8. Strahlereinheit (100) nach zumindest einem der Ansprüche 6 bis 7,
**dadurch gekennzeichnet, dass**
das Gestänge (90) in Abhängigkeit der Länge der Halterung einen oder mehrere Isoliersterne (92) als Stabilisierungsvorrichtungen aufweist, die über die Länge der Halterung verteilt angeordnet sind, wobei der Isolierstern (92) parallel orientierte Hülsen (93) entsprechend der Anzahl der parallelen Rohre (9) oder Rohranordnungen des Gestänges (90) aufweist, und die Hülsen (93) einen Innendurchmesser zur Aufnahme des Rohrabschnitts (91') aufweisen und durch ein Verbindungsstück (94) in einer für das Gestänge (90) vorbestimmten Anordnung und Abstand voneinander gehalten werden.

9. Strahlereinheit (100) nach zumindest einem der Ansprüche 1 bis 8,
**dadurch gekennzeichnet, dass**
die Strahlereinheit (100) zumindest zwei Zentrierelemente (101) zur zentrierenden Halterung des Strahlers (10) in einem Hüllrohr (20) aufweist, wobei das Zentrierelement (101) einen Scheibengrundkörper (120) mit zumindest einer axial-zentralen Bohrung (4) für einen Strahlersockel (11) und zumindest zwei, bevorzugt zumindest drei, exzentrischen Bohrungen (41) für das Gestänge (90) aufweist, und wobei der Durchmesser des Scheibengrundkörpers (120) kleiner ist als ein Innendurchmesser des Hüllrohrs (200), und das Zentrierelement (101) zumindest ein elastisches Federelement (3) aufweist, das sich zumindest abschnittsweise an einem Außenumfang des Scheibengrundkörpers (120) erstreckt und eine radiale Rückstellkraft bereitstellt.

10. Strahlereinheit (100) nach zumindest einem der Ansprüche 1 bis 9,
**dadurch gekennzeichnet, dass**
jeweils zumindest ein Zentrierelement (101) beiderseits des Strahlers angeordnet ist und in Abhängigkeit der Länge der Halterung gegebenenfalls noch weitere Zentrierelemente (101) entlang des Gestänges (90) angeordnet sind.

11. Strahlereinheit (100) nach zumindest einem der Ansprüche 6 bis 10,
**dadurch gekennzeichnet, dass**
ein erstes der Rohre (9) oder Rohranordnungen, das einen Phasenleiter bereitstellt, sich bis zu dem Zentrierelement (101), das kopfteilseitig benachbart zu dem Strahler (10) angeordnet ist, zum elektrischen Anschluss des Strahlers (10) an einem ersten, kopfteilnahen Strahlersockel (11) erstreckt,
und die weiteren Rohre (9) oder Rohranordnungen, die Neutralleiter bereitstellen, sich bis zu dem Zentrierelement (101) erstrecken, das kopfteilfern benachbart zu dem Strahler (10) angeordnet ist, wobei in der axial-zentralen Bohrung (4) des kopfteilfernen Zentrierelements (101) ein Anschlusselement (5) für den zweiten, kopfteilfernen Strahlersockel (11) vorliegt und das Anschlusselement (5) mit den Rohren (9) oder Rohranordnungen, die die Neutralleiter bereitstellen, elektrisch kontaktierend verbunden ist.

12. Strahlereinheit (100) nach Anspruch 11,
**dadurch gekennzeichnet, dass**
der elektrischer Anschluss des ersten Rohrs (9) oder Rohranordnung, die den Phasenleiter bereitstellt, mit dem ersten, kopfteilnahen Strahlersockel (11) durch eine isolierte Leitung (96) mit Überlänge in Bezug auf den Abstand des Rohrendes des ersten Rohrs (9) oder Rohranordnung zum ersten, kopfteilnahen Strahlersockel (11) bereitgestellt wird, wobei gegebenenfalls ein zusätzlicher Isolierstern (92') vorgesehen ist, der zwei Hülsen (93) für die weiteren Rohre und eine axial-zentrierte Durchtrittsöffnung (94') in dem Verbindungsstück (94) für den ersten, kopfteilnahen Strahlersockel (11) aufweist.

## Claims

1. A radiation unit (100) comprising a head element (1) and a retainer for a radiator (10), wherein the head element (1) has a basic body (1a) that is remote from the radiator, is made of an electrically insulating material, and has a protective gas supply device, and
wherein the radiation unit (100) has a linkage (90) as a retainer for the radiator (10), said linkage (90) consisting of a plurality of tubes (9) or of a plurality of rods and of at least one tube (9) made of an electrically conducting material,
wherein the electrical connecting lines required for the radiator (10) are formed through at least a portion of the tubes (9) or rods, wherein the respective tubes and/or rods are in contact with the electrical connecting device at one of their ends and, at the other one of their ends, provide the electrical connection of the radiator (10),
and wherein at least one of the tubes (9) is in fluid communication with the protective gas supply device at one of its ends and, at the other one of its ends, provides the protective gas supply to the radiator (10),
**characterised in that**
the head element (1) has a connecting body (1b) that is proximate to the radiator, is made of an electrically insulating material, is detachably connected to the basic body (1a) and has parallel-oriented access openings (15', 17') for the tubes (9) or for the rods and the tube (9), said access openings (15', 17') being detachably mounted to the connecting body (1b), and
the basic body (1a) has holes (15, 17) which are aligned with the access openings (15', 17') in the connecting body (1b),
wherein the electrical contact of the respective tubes (9) and/or rods forming the electrical connecting lines with the electrical connecting device, and the fluid communication of the at least one tube (9) provided for supplying the radiator (10) with protective gas with the protective supply device are provided by the connection of the basic body (1a) to the connecting body (1b), wherein connecting holes (19, 19') extend through the basic body (1a) from at least one of the sides of the basic body (1a) to the holes (15, 17) in a region remote from the connecting body (1b), wherein an electrical contact element (28) of the electrical connecting device is present in each of the connecting holes (19, 19') for each of the connecting lines required for the radiator (10), and
a push-on sleeve (20) is present in each of the holes (15, 17), which is contacted by the electrical contact element (28) and is respectively connected to one of the tubes (9) received in the access openings (15', 17') of the connecting body (1b) either directly or via a connecting plug (21),
and wherein at least one hole (17) of the holes (15, 17) in the basic body (1a) is concentrically surrounded by an annular blind hole (16) the depth of which is less than a distance from the connecting holes (19, 19'), wherein a further connecting hole (18) from at least one of the sides of the basic body (1a) ends in each of said annular blind holes (16), wherein the further connecting hole (18) and the annular blind hole (16) provide the protective gas supply device.

2. The radiation unit (100) according to claim 1,
**characterised in that**
the push-on sleeve (20) has a sleeve section (20') the open end of which faces the connecting body (1b) and a contact section (20") which extends at least to the connecting hole (19, 19'), wherein
the connecting plug (21) has a receiving section (23) for one of the tubes (9) and a pin section (22) which can be received or is received in the sleeve section (20') of the push-on sleeve (20).

3. The radiation unit (100) according to claim 1 or 2,
**characterised in that**
in the connecting body (1b), the access opening (17') widens toward the basic body (1a) in a stepped manner, wherein the first stepped opening (17") is formed for receiving the receiving section (23) of the connecting plug (21), wherein the pin section (22) of the connecting plug (21) projects beyond the connecting body (1b), and the diameter of a second stepped opening (16') corresponds to the outside diameter of the annular blind hole (16) in the basic body (1a) and is aligned therewith when the basic body (1a) and the connecting body (1b) are connected.

4. The radiation unit (100) according to at least any one of claims 1 to 3,
**characterised in that**
the receiving section (23) of the connecting plug (21) has radial connecting holes (24) to a cavity (26) present in the connecting plug (21), said cavity (26) being connected to an orifice of the tube (9) received in the receiving section (23).

5. The radiation unit (100) according to at least any one of claims 1 to 4,
**characterised in that**
the detachable connection of the basic body (1a) to the connecting body (1b) is provided by pens, screws or stud bolts (95) which are located in openings (14') of the connecting body (1b) and have a length for penetrating the basic body (1a) that has access openings (14) aligned with the openings (14').

6. The radiation unit (100) according to at least any one of claims 1 to 5,
**characterised in that**
each tube (9) is formed by a tube arrangement consisting of a plurality of tubes (9) that are arranged in series and have at their ends engagement elements corresponding to each other, preferably an external thread section (9') at one end and a corresponding internal thread section at the other end, with the result that a desired length of the retainer can be adjusted with a tube arrangement by connecting, preferably screwing, a plurality of tubes (9) to each other.

7. The radiation unit (100) according to at least any one of claims 1 to 6,
**characterised in that**
each tube (9) of the linkage (90) is surrounded by an isolator tube (91) which preferably has a tube section (91') and a terminal widened sleeve section (91"), with the result that a plurality of isolator tubes (91) can be fitted together to form an isolator tube arrangement depending on the desired position length of the retainer, wherein an end section of the tube section (91') of a first isolator tube (91) is received in the sleeve section (91") of a second isolator tube (91).

8. The radiation unit (100) according to at least any one of claims 6 to 7,
**characterised in that**
depending on the length of the retainer, the linkage (90) has one or more isolating stars (92) as stabilisation devices which are arranged distributed across the length of the retainer, wherein the isolating star (92) has parallel-oriented sleeves (93) corresponding to the number of the parallel tubes (9) or tube arrangements of the linkage (90) and **in that** the sleeves (93) have an inside diameter for receiving the tube section (91') and are kept spaced apart from each other with an arrangement and distance predetermined for the linkage (90) by means of a connecting piece (94).

9. The radiation unit (100) according to at least any one of claims 1 to 8,
**characterised in that**
the radiation unit (100) has at least two centring elements (101) for centrally retaining the radiator (10) in a jacket tube (20), wherein the centring element (101) has a disc basic body (120) with at least one axially central hole (4) for a radiator base (11) and at least two, preferably three, eccentric holes (41) for the linkage (90), and wherein the diameter of the disc basic body (120) is less than an inside diameter of the jacket tube (200), and the centring element (101) has at least one elastic spring element (3) which extends along an outer perimeter of the disc basic body (120) at least in certain regions and provides a radial restoring force.

10. The radiation unit (100) according to at least any one of claims 1 to 9,
**characterised in that**
at least one centring element (101) is arranged on each side of the radiator, and **in that** further centring elements (101) are arranged along the linkage (90) depending on the length of the retainer, if necessary.

11. The radiation unit (100) according to at least any one of claims 6 to 10,
**characterised in that**
a first one of the tubes (9) or tube arrangements, which provides a phase conductor, extends to the centring element (101) that is arranged adjacent to the radiator (10) on the side of the head element, for electrically connecting the radiator (10) to a first radiator base (11) proximate to the head element,
and the further tubes (9) or tube arrangements, which provide neutral conductors, extend to the centring element (101) that is arranged adjacent to the radiator (10) remote from the head element, wherein a connecting element (5) for the second radiator base (11) remote from the head element is present in the axially central hole (4) of the centring element (101) remote from the head element, and **in that** the connecting element (5) is connected to the tubes (9) or tube arrangements providing the neutral conductors in an electrically contacting manner.

12. The radiation unit according to claim 11,
**characterised in that**
the electrical connection of the first tube (9) or tube arrangement providing the phase conductor to the first radiator base (1) proximate to the head element is provided by an insulated line (96) having an excess length with regard to the distance of the tube end of the first tube (9) or tube arrangement from the first radiator base (11) proximate to the head element, wherein, if necessary, an additional isolating star (92') is provided which has two sleeves (93) for the further tubes and an axially centred access opening (94') in the connecting piece (94) for the first radiator base (11) proximate to the head element.

## Revendications

1. Module d'émetteur (100) avec une partie de tête (1) et une fixation pour un émetteur (10), dans lequel la partie de tête (1) présente un corps de base (1a) éloigné de l'émetteur en un matériau électriquement isolant qui présente un dispositif de raccordement électrique et un dispositif d'amenée de gaz protecteur, et dans lequel le module d'émetteur (100) présente une tringlerie (90) en tant que fixation pour l'émetteur (10) qui se compose d'une pluralité de tubes (9) ou d'une pluralité de tiges et d'au moins un tube (9) en un matériau électriquement conducteur,
dans lequel les lignes de raccordement électriques nécessaires pour l'émetteur (10) sont formées à travers au moins une partie des tubes (9) ou tiges, dans lequel les tubes et/ou tiges respectifs sont en contact à une extrémité avec le dispositif de raccordement électrique et mettent à disposition à leur autre extrémité le raccordement électrique de l'émetteur (10),
et dans lequel au moins un des tubes (9) est en communication fluidique à une extrémité avec le dispositif d'amenée de gaz protecteur et met à disposition à l'autre extrémité l'amenée de gaz protecteur vers l'émetteur (10),
**caractérisé en ce que**
la partie de tête (1) présente un corps de raccordement (1b) proche de l'émetteur en un matériau électriquement isolant qui est connecté de manière amovible au corps de base (1a) et qui présente des ouvertures de passage orientées parallèlement (15', 17') pour les tubes (9) ou pour les tiges et le tube (9) qui sont fixés de manière amovible au corps de raccordement (1b), et
le corps de base (1a) présente des alésages (15, 17) qui sont alignés avec les ouvertures de passage (15', 17') dans le corps de raccordement (1b),
dans lequel le contact électrique des tubes (9) et/ou tiges respectifs qui forment les lignes de raccordement électriques avec le dispositif de raccordement électrique et la communication fluidique de l'au moins un tube (9) qui est prévu pour l'amenée de gaz protecteur vers l'émetteur (10) avec le dispositif d'amenée de gaz protecteur sont mis à disposition par la connexion du corps de base (1a) au corps de raccordement (1b), dans lequel des alésages de connexion (19, 19') s'étendent à travers le corps de base (1a) d'au moins un des côtés du corps de base (1a) aux alésages (15, 17) dans une région située à l'écart du corps de raccordement (1b), dans lequel
un élément de contact électrique (28) du dispositif de raccordement électrique est à chaque fois présent dans les alésages de connexion (19, 19') pour chacune des lignes de raccordement nécessaires pour l'émetteur (10) et
une prise femelle (20) est à chaque fois présente dans les alésages (15, 17), laquelle est contactée par l'élément de contact électrique (28) et est connectée directement ou par le biais d'une fiche de connexion (21) à chaque fois à un des tubes (9) qui sont reçus dans les ouvertures de passage (15', 17') du corps de raccordement (1b), et dans lequel au moins un alésage (17) des alésages (15, 17) est entouré de manière concentrique dans le corps de base (1a) par un alésage à anneau borgne (16) dont la profondeur est inférieure à un écart aux alésages de connexion (19, 19') et dans lequel un autre alésage de connexion (18) débouche à chaque fois d'au moins un des côtés du corps de base (1a), dans lequel l'autre alésage de connexion (18) et l'alésage à anneau borgne (16) mettent à disposition le dispositif d'amenée de gaz protecteur.

2. Module d'émetteur (100) selon la revendication 1,
**caractérisé en ce que**
la prise femelle (20) présente une section de manchon (20') dont l'extrémité ouverte est dirigée vers le corps de raccordement (1b) et une section de contact (20") qui s'étend au moins jusqu'à l'alésage de connexion (19, 19'), dans lequel la fiche de connexion (21) présente une section de réception (23) pour un des tubes (9) et une section de tourillon (22) qui peut être reçue ou est reçue dans la section de manchon (20') de la prise femelle (20).

3. Module d'émetteur (100) selon la revendication 1 ou 2,
**caractérisé en ce que**
l'ouverture de passage (17') s'élargit de manière échelonnée dans le corps de raccordement (1b) vers le corps de base (1a), dans lequel une première ouverture échelonnée (17") est réalisée pour la réception de la section de réception (23) de la fiche de connexion (21), dans lequel la section de tourillon (22) de la fiche de connexion (21) fait saillie au-delà du corps de raccordement (1b), et une seconde ouverture échelonnée (16') correspond en diamètre au diamètre externe de l'alésage à anneau borgne (16) dans le corps de base (1a) et est alignée avec celui-ci lorsque le corps de base (1a) et le corps de raccordement (1b) sont connectés.

4. Module d'émetteur (100) selon au moins une des revendications 1 à 3,
**caractérisé en ce que**
la section de réception (23) de la fiche de connexion (21) présente des alésages de connexion radiaux (24) vers un espace creux (26) présent dans la fiche de connexion (21) qui est connecté à une embouchure du tube (9) qui est reçu dans la section de réception (23).

5. Module d'émetteur (100) selon au moins une des revendications 1 à 4,
**caractérisé en ce que**
la connexion amovible du corps de base (1a) au corps de raccordement (1b) est mise à disposition par des chevilles, vis ou tirants (95) qui sont fixés dans des ouvertures (14') du corps de raccordement (1b) et présentent une longueur pour la pénétration du corps de base (1a) qui présente des ouvertures de passage (14) s'alignant avec les ouvertures (14').

6. Module d'émetteur (100) selon au moins une des revendications 1 à 5,
**caractérisé en ce que**
chaque tube (9) est formé par un agencement de tubes constitué de plusieurs tubes juxtaposés (9) qui présentent aux extrémités des éléments d'engrènement correspondants les uns aux autres, de préférence à une extrémité une section de filetage externe (9') et à l'autre extrémité une section de filetage interne correspondante de sorte qu'une longueur souhaitée de la fixation avec un agencement de tubes peut être réglée par connexion, de préférence vissage de plusieurs tubes (9).

7. Module d'émetteur (100) selon au moins une des revendications 1 à 6,
**caractérisé en ce que**
chaque tube (9) de la tringlerie (90) est entouré par un tube isolant (91) qui présente de préférence une section de tube (91') et une section de manchon évasée terminale (91") de sorte que plusieurs tubes isolants (91) peuvent être assemblés en un agencement de tubes isolants en fonction de la longueur souhaitée de la fixation, dans lequel une section d'extrémité de la section de tube (91') d'un premier tube isolant (91) est reçue dans la section de manchon (91") d'un second tube isolant (91).

8. Module d'émetteur (100) selon au moins une des revendications 6 à 7,
**caractérisé en ce que**
la tringlerie (90) présente une ou plusieurs étoiles isolantes (92) en tant que dispositifs de stabilisation en fonction de la longueur de la fixation qui sont disposées de manière répartie sur la longueur de la fixation, dans lequel l'étoile isolante (92) présente des manchons orientés parallèlement (93) correspondant au nombre des tubes parallèles (9) ou agencements de tubes de la tringlerie (90), et les manchons (93) présentent un diamètre interne pour la réception de la section de tube (91') et sont maintenus par une pièce de connexion (94) dans un agencement prédéterminé pour la tringlerie (90) et à un écart les uns des autres.

9. Module d'émetteur (100) selon au moins une des revendications 1 à 8,
**caractérisé en ce que**
le module d'émetteur (100) présente au moins deux éléments de centrage (101) pour la fixation centrée de l'émetteur (10) dans un tube d'enveloppe (20), dans lequel l'élément de centrage (101) présente un corps de base de disque (120) avec au moins un alésage axial-central (4) pour un socle d'émetteur (11) et au moins deux, de préférence au moins trois, alésages excentriques (41) pour la tringlerie (90), et dans lequel le diamètre du corps de base de disque (120) est inférieur à un diamètre interne du tube d'enveloppe (200), et l'élément de centrage (101) présente au moins un élément de ressort élastique (3) qui s'étend au moins par section à une périphérie externe du corps de base de disque (120) et met à disposition une force de rappel radiale.

10. Module d'émetteur (100) selon au moins une des revendications 1 à 9,
**caractérisé en ce que**
au moins un élément de centrage (101) est à chaque fois disposé des deux côtés de l'émetteur et d'autres éléments de centrage (101) sont éventuellement encore disposés le long de la tringlerie (90) en fonction de la longueur de la fixation.

11. Module d'émetteur (100) selon au moins une des revendications 6 à 10,
**caractérisé en ce que**
un premier des tubes (9) ou agencements de tubes qui met à disposition un fil de phase s'étend jusqu'à l'élément de centrage (101) qui est disposé du côté de la partie de tête de manière adjacente à l'émetteur (10) pour le raccordement électrique de l'émetteur (10) à un premier socle d'émetteur (11) proche de la partie de tête,
et les autres tubes (9) ou agencements de tubes qui mettent à disposition des fils neutres s'étendent jusqu'à l'élément de centrage (101) qui est disposé de manière éloignée de la partie de tête de manière adjacente à l'émetteur (10), dans lequel un élément de raccordement (5) pour le second socle d'émetteur (11) éloigné de la partie de tête est présent dans l'alésage axial-central (4) de l'élément de centrage (101) éloigné de la partie de tête, et l'élément de raccordement (5) est connecté par contact électrique aux tubes (9) ou agencements de tubes qui mettent à disposition les fils neutres.

12. Module d'émetteur (100) selon la revendication 11,
**caractérisé en ce que**
le raccordement électrique du premier tube (9) ou agencement de tubes qui met à disposition le fil de phase au premier socle d'émetteur (11) proche de la partie de tête est mis à disposition par une ligne isolée (96) avec une surlongueur par rapport à l'écart de l'extrémité de tube du premier tube (9) ou agencement de tubes par rapport au premier socle d'émetteur (11) proche de la partie de tête, dans lequel une étoile isolante supplémentaire (92') est éventuellement prévue, laquelle présente deux manchons (93) pour les autres tubes et une ouverture de passage à centrage axial (94') dans la pièce de connexion (94) pour le premier socle d'émetteur (11) proche de la partie de tête.
